(19) Europäisches Patentamt
European Patent Office
Office européen des brevets

(11) EP 4 575 874 A1

(12) EUROPEAN PATENT APPLICATION

(43) Date of publication:
**25.06.2025 Bulletin 2025/26**

(21) Application number: **23218407.7**

(22) Date of filing: **20.12.2023**

(51) International Patent Classification (IPC):
***G06F 30/17*** *(2020.01)* ***G06F 30/28*** *(2020.01)*
***G16C 20/10*** *(2019.01)* *G06F 111/08 (2020.01)*
*G06F 111/10 (2020.01)* *G06F 113/08 (2020.01)*

(52) Cooperative Patent Classification (CPC):
**G06F 30/28; G06F 30/17;** G06F 2111/08;
G06F 2111/10; G06F 2113/08

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Terra Quantum AG**
**9000 St. Gallen (CH)**

(72) Inventors:
- **Melnikov, Artem A.**
  **9000 St. Gallen (CH)**
- **Perelshtein, Michael R.**
  **9000 St. Gallen (CH)**

(74) Representative: **Lucke, Andreas**
**Boehmert & Boehmert**
**Anwaltspartnerschaft mbB**
**Pettenkoferstrasse 22**
**80336 München (DE)**

(54) **COMPUTER-IMPLEMENTED METHOD FOR SIMULATING FLUID FLOW, COMPUTER-IMPLEMENTED METHOD FOR DESIGNING A MIXING REACTOR, MIXING REACTOR AND COMPUTER-IMPLEMENTED METHOD FOR CONTROLLING A MIXING REACTOR, AND CORRESPONDING DATA PROCESSING DEVICE, COMPUTER PROGRAM AND COMPUTER-READABLE MEDIUM**

(57) The invention relates to a computer-implemented method for simulating fluid flow, wherein a computational grid (20) having grid points (30) is provided for computing a distribution function (f, 2) of the fluid flow and/or a distribution function (f, 2) of a mixture of at least two species at the grid points (30), the method comprising a Lattice Boltzmann method having a collision step, wherein at each grid point (30) a difference of the distribution function (f) to an equilibrium solution ($f^{eq}$) is computed; and a streaming step, wherein the distribution function (f) at each grid point (30) is updated according to the streaming step; and/or the method comprising solving a Lattice Boltzmann equation for the distribution function (f, 2), characterized in that the distribution function (f, 2)is represented in a tensor-train format, and/or all operations carried out for computing the distribution function (f, 2) are carried out with the distribution function (f, 2) in the tensor-train format, the distribution function (f, 2) and/or the tensor train format comprising at least one tensor train (3), the tensor train (3) having at least one tensor train core (4). The invention further relates to a method for designing a mixing reactor, a mixing reactor, a method for controlling a mixing reactor, and a corresponding data processing device, corresponding computer program and corresponding computer-readable medium.

Fig. 5

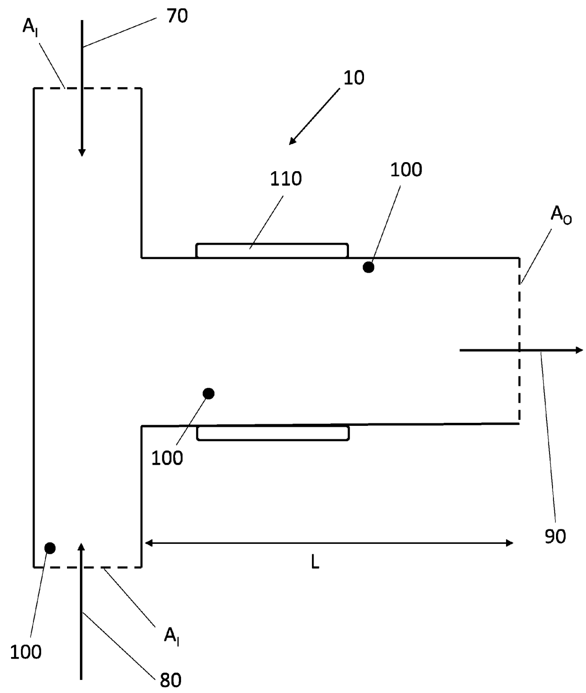


EP 4 575 874 A1

## Description

### Field of the Invention

The invention relates to a computer-implemented method for simulating fluid flow, a computer-implemented method for designing a mixing reactor, a mixing reactor, and computer-implemented method for controlling a mixing reactor. The invention further relates to a data processing device configured to perform the computer-implemented methods, a corresponding computer program, and a corresponding computer-readable medium.

### Background

Known methods for simulating fluid flow comprise Lattice Boltzmann methods (LBM) and/or solving Lattice Boltzmann equations. As is well known, the Lattice Boltzmann Method is a discretization on a uniform lattice of the basic kinetic Boltzmann equation. Both Lattice Boltzmann methods and Lattice Boltzmann equations are frequently used in computational fluid dynamics.

It is well known that the Navier-Stokes equations may be derived from Lattice Boltzmann methods, , and/or the Navier-Stokes equations may be reproduced by Lattice Boltzmann methods, cf. e.g. Chen and Doolen, "Lattice boltzmann method for fluid flows", Annual review of fluid mechanics, 30(1):329-364, 1998 or Benzi, Succi and Vergassola, "The lattice Boltzmann equation: theory and applications", Physics reports (Review Section of Physics Letters) 222, No. 3, 145-197, 1992.

For complex flow geometries and/or high Reynolds number flows or mixtures, a fine mesh (computational grid) is required, which in turn implies a large number of grid points. At the same time, velocities discretization occurs in such a way that in one time step the particles can move exactly and/or only to the neighboring vertexes of the lattice or stay in place.

Both of these effects mean that a lot of memory is needed. Usually, workstations do not have enough memory for this purpose, so that computer clusters or distributed computing systems with many distributed nodes are employed. This means that such simulations are mostly restricted to academic purposes, and/or where access to such computer systems is available.

Moreover, the computational speed can be reduced, since a lot of communication between nodes may be required due to the limited amount of memory per node. That is, a lot of network traffic can occur, and/or a lot of data may have to be shared or communicated between nodes. Specifically, a node may need to wait for one or more other nodes before it can carry on with its own computations. In some scenarios, the total amount of memory may be not sufficient, such that some of the data would be dumped to storage, and/or swapping of memory occurs, which significantly reduces speed.

### Overview

The present invention overcomes these obstacles. By requiring significantly less memory than the methods of the art, the methods according to the invention may be faster, more accurate and/or allow for more complex geometries, and/or higher Reynolds numbers, than methods known from the art.

It is therefore an object of the invention to provide a method for simulating fluid flow, a method for designing a mixing reactor and a method for controlling a mixing reactor which need less memory, which may be faster and/or which may be used on more conventional computing devices.

The method for designing a mixing reactor may allow for more complex geometries of the reactor to be designed, with higher accuracy and in a reasonable time frame. Further, the method for designing a mixing reactor may, in some embodiments, be suitable to be carried out by workstations or relatively small distributed systems, which may be cheaper, more accessible and/or more feasible compared to known methods. Since the computational speed may be increased, different designs may be more easily compared.

The method for controlling a mixing reactor may allow for better control and/or more accurate mixing. Since the computational speed may be increased, in some embodiments it can be possible to use results of the method and/or generate or update control commands when mixing in the controlled reactor takes place, such that the control command may be generated and/or updated "live" and/or during operation of the mixing reactor. In some embodiments, it can be possible to generate and/or adapt the control command such as to react, and/or in response to, the real mixing and/or flow in the mixing reactor. The method allows for a good mixing quality and/or quality of mixing product, and/or to achieve, or be at least close to a, given or desired quality.

Another object of the invention is to provide a mixing reactor designed by the method for designing a mixing reactor. Yet another object of the invention is to provide a data processing device to perform one, several or all methods of the invention, to provide a computer program comprising instructions to perform one, several or all methods of the invention, and a computer-readable medium comprising instructions to perform one, several or all methods of the invention.

This object is met by a method for simulating fluid flow according to claim 1, a method for designing a mixing reactor according to claim 5, a mixing reactor according to claim 7, a method for controlling a mixing reactor according to claim 8, a data processing device according to claim 13, a computer program according to claim 14 and a computer-readable medium according to claim 15. Particularly preferred embodiments are the subject-matter of the dependent claims.

A first aspect of the invention relates to a computer-implemented method for simulating fluid flow, wherein a computational grid having grid points is provided for computing a distribution function of the fluid flow and/or a distribution function of a mixture of at least two species at the grid points, the method comprising a Lattice Boltzmann method having

- a collision step, wherein at each grid point a difference of the distribution function to an equilibrium solution is computed; and

- a streaming step, wherein the distribution function at each grid point is updated according to the streaming step;

and/or the method comprising solving a Lattice Boltzmann equation for the distribution function, wherein the distribution function is represented in a tensor-train format, and/or all operations carried out for computing the distribution function are carried out in the tensor-train format.

The distribution function and/or the tensor train format comprise at least one tensor train, the tensor train having at least one tensor train core. The distribution function may be or comprise a tensor. The tensor-train format may comprise at least one tensor-train.

The distribution function can be a probability density function. The distribution function can be a probability density function of finding a fluid particle, e.g. of finding a fluid particle at a grid point. The distribution function can be a fluid particle density.

In some embodiments, the distribution function can be a probability density function of finding a particle of a species of a mixture, e.g. of finding a particle of a species at a grid point. The distribution function can be a species density.

The equilibrium solution can be given by

$$f_k^{eq,\alpha} = w_k \rho^\alpha \left[ 1 + \frac{3}{c^2} \mathbf{e}_k \cdot \mathbf{u} + \frac{9}{2c^4} \left( \mathbf{e}_k \cdot \mathbf{u} \right)^2 + \frac{3}{2c^2} \mathbf{u} \cdot \mathbf{u} \right],$$

where $e_k$ can be a lattice velocity in direction k, $w_k$ can be weighting coefficients, $\rho^a$ can be the fluid density of species $\alpha$, u can be a macroscopic velocity, and/or c can be a lattice speed. The superscript $\alpha$ can indicate a species. It may be provided that the fluid density can be determined by

$$\rho^\alpha \left( \mathbf{x}, t \right) = \sum_k f_k \left( \mathbf{x}, t \right).$$

The macroscopic velocity can be determined by

$$\rho^\alpha \left( \mathbf{x}, t \right) \mathbf{u}^\alpha \left( \mathbf{x}, t \right) = \sum_k \mathbf{e}_k f_k^\alpha \left( \mathbf{x}, t \right).$$

In some embodiments, the flow can comprise a mixture of at least two species a. A distribution function, or the distribution function, can be computed for each species a. In some embodiments, the flow may comprise of a single species, e.g. when the distribution function of a fluid flow without mixing is simulated. In some embodiments and/or in some of these cases, the index $\alpha$ can be dropped from the equations, and/or be implied.

When a mixture of at least two species is simulated, it may be provided that the macroscopic velocity can be determined by

$$\mathbf{u}(\mathbf{x},t) = \frac{\sum_k \rho^\alpha(\mathbf{x},t)\,\mathbf{u}^\alpha(\mathbf{x},t)}{\sum_k \rho^\alpha(\mathbf{x},t)}.$$

This may amount to density-weighting the respective macroscopic velocities of the respective species.

It can be provided that in the collision step, an intermediary distribution function $\tilde{f}_k^\alpha$ can be given and/or computed by

$$\tilde{f}_k^\alpha(\mathbf{x},t) = -\frac{\Delta t}{\tau}\left(f_k^\alpha(\mathbf{x},t) - f_k^{eq,\alpha}(\mathbf{x},t)\right).$$

In the streaming step, the distribution function $f_k^a$ at the grid points can be updated by

$$f_k^\alpha(\mathbf{x}+\mathbf{e}_k\Delta t, t+\Delta t) = \tilde{f}_k^\alpha(\mathbf{x},t).$$

In some embodiments, it may be provided that the collision step can correspond to the r.h.s and the streaming step to the l.h.s. of

$$f_k^\alpha(\mathbf{x}+\mathbf{e}_k\Delta t, t+\Delta t) - f_k^\alpha(\mathbf{x},t) = -\frac{\Delta t}{\tau}\left(f_k^\alpha(\mathbf{x},t) - f_k^{eq,\alpha}(\mathbf{x},t)\right).$$

In some embodiments, the Lattice Boltzmann equation may be given by

$$\frac{\partial f_k}{\partial t}(\mathrm{x},t) + \mathrm{e}_k \cdot \nabla f_k(\mathrm{x},t) = -\frac{1}{\tau}\left(f_k(\mathbf{x},t) - f_k^{eq}(\mathbf{x},t)\right).$$

Here, the index α might be omitted and/or implied. The Lattice Boltzmann equation may be different and/or contain additional terms, depending on the application and/or flow or mixture to be simulated. In some embodiments, the Lattice Boltzmann equation may comprise at least one term for external forces, and/or temperature effects, as is obvious and/or known to the person skilled in the art.

Solving the Lattice Boltzmann equation may comprise solving a linear system of equations

$$\underbrace{\left(\left(\frac{1}{\Delta t} + \frac{1}{\tau}\right) I + \mathbf{e}\cdot\nabla\right)}_{A} f^{i+1} = \underbrace{\frac{f^i}{\Delta t} + \frac{f^{eq}(f^i)}{\tau}}_{b}.$$

In some embodiments, solving the Lattice Boltzmann equation may comprise solving a linear system of equations

$$\underbrace{\left(\frac{1}{\Delta t}A_t + \frac{1}{\Delta x}A_{\mathbf{x}} + \frac{1}{\tau}I\right)}_{A} f = \frac{1}{\tau}f^{eq}(f) + \frac{1}{\Delta t}f(t=0)\otimes e_1,$$

The tensor train may be or comprise a quantized tensor train. The tensor train cores of the tensor train may be reshaped and/or refined into quantized tensor train cores.

The tensor-train can have tensor train cores respectively corresponding to grid coordinates. Alternatively or

additionally, the tensor-train can have tensor train cores corresponding to velocity components of the flow, species and/or mixture.

In some embodiments, the tensor-train can have at least one tensor train core corresponding to the species a. Alternatively or additionally, in some embodiments, a tensor-train is provided for each species a.

In some embodiments, it may be provided that the tensor train format comprises a tensor train which has an index for the species. In some embodiments, a tensor $f(\mathbf{x}, \mathbf{v}, \alpha)$ and/or $f(\mathbf{x}, \mathbf{v}, t, a)$ may be provided. In some embodiments, a tensor may be provided for the distribution function which comprises at least two, multiple and/or all species. In some embodiments, a single tensor may be provided for the distribution function which comprises all species.

Alternatively, it may be provided that at least two species, and/or each species, has a respective tensor train and/or is given its own tensor train. In some embodiments, a respective tensor $f(x, v)$ and/or $f(\mathbf{x}, \mathbf{v}, t)$ may be provided for each species.

In some embodiments, the notation $f_k^{\alpha}(\mathbf{x}, \mathbf{v}, \mathrm{t})$ may correspond to, or be equal to, the notation $f(x_1, x_2, v_x, v_y, t, a)$ in case of a two-dimensional flow and/or two-dimensional mixture. In some embodiments, the notation $f_k^{\alpha}(\mathbf{x}, \mathbf{v}, \mathrm{t})$ may correspond to, or be equal to, the notation $f(x_1, x_2, x_3, v_x, v_y, v_z, t, a)$ in case of a three-dimensional flow and/or three-dimensional mixture.

Another aspect of the invention relates to a computer-implemented method for designing a mixing reactor for mixing at least two species, wherein the method comprises providing an initial mixing reactor geometry having a computational grid with grid points, the method comprising the steps:

a) simulating a mixing of at least two species, and/or a flow in and/or through the mixing reactor, via any of the methods according to the invention, and determining a characteristic property of the mixture and/or fluid flow;

b) modifying the mixing reactor geometry and/or grid points, repeating the simulating using the modified mixing reactor geometry and/or grid points, and determining the characteristic property of the mixture;

c) comparing the characteristic property to a target value of the characteristic property;

d) iteratively repeating the steps b) and c), and optimizing the mixing reactor geometry and/or the computational grid, such that the mixing reactor geometry and/or the computational grid is optimized with respect to the characteristic property, and/or until a given accuracy of the characteristic property to the target value is achieved.

The characteristic property can be or can comprise at least one of a mixing fraction, a species concentration, a characteristic mixing time, a characteristic residence time and/or a mixing progress. In some embodiments, the characteristic property can be or comprise a property derived from and/or related to at least one of a mixing fraction, a species concentration, a characteristic mixing time, a characteristic residence time and/or a mixing progress.

Modifying the mixing reactor geometry can comprise moving a boundary and/or changing the shape of the computational grid. Modifying the grid points can comprise adding or removing grid points from the computational grid. Modifying the grid points can comprise coarsening or refining the computational grid.

Another aspect of the invention relates to a mixing reactor for mixing at least two species, the mixing reactor having a mixing reactor geometry, which is determined via a method of designing a mixing reactor according to the invention. In some embodiments, the geometry is or comprises an interior geometry of the mixing reactor.

Another aspect of the invention relates to a computer-implemented method for controlling a mixing reactor, the method comprising:

- simulating a mixing of at least two species, and/or a flow in and/or through the mixing reactor, via the method of any of the methods according to the invention, wherein the computational grid corresponds to a geometry of the mixing reactor;
- generating and/or adapting at least one control command for the mixing reactor based on the simulation.

The control command can comprise a command for controlling an inflow of at least one flow into the mixing reactor and/or an outflow of at least one flow out of the reactor, optionally a velocity, volume flow rate and/or mass flow rate. Alternatively or additionally, the control command can comprise a command for controlling an agitator, optionally an angular velocity or frequency. Alternatively or additionally, the control command can comprise a command for controlling temperature control unit, such as e.g. a heater and/or cooler. The control command can comprise a command to control or set a cooling or heating temperature.

The method can comprise measuring a fluid property, optionally a velocity and/or a species concentration, at at least one measure point in the mixing reactor and comparing the measured fluid property with a corresponding fluid property obtained from the simulation. The control command can be generated, adapted and/or modified based on the comparison. The fluid property can be measured using a sensor. The sensor can be located at or near the measure point. The measure point can correspond to a grid point of the computational grid.
The control command can be generated and/or updated while or when mixing the at least two species in the mixing reactor. The mixing reactor can be controlled by the control command.
It may be provided that after generating and/or updating the control command, the simulation of the mixing can be repeated. When repeating the simulation, the distribution function can be computed taking the generated and/or updated control command into account. In some embodiments, when repeating the simulation, the distribution function can be computed taking at least one of forces, flow velocities and/or boundary conditions imposed and/or exerted by the control command into account.
Another aspect of the invention relates to a data processing device comprising a processor configured to perform the steps of any of the methods according to the invention.
Another aspect of the invention relates to a computer program comprising instructions which, when the program is executed by a computer, cause the computer to carry out the steps of any method according to the invention.
Another aspect of the invention relates to a computer-readable medium comprising instructions which, when executed by a computer, cause the computer to carry out the steps of any method according to the invention.

## Brief Description of the Figures

The invention is further detailed with reference to the following figures:

Fig. 1: an exemplary grid;

Fig. 2: an exemplary tensor train decomposition;

Fig. 3: another exemplary tensor train decomposition;

Fig. 4: runtime of an exemplary embodiment of a method according to the invention compared to a method known from prior art, for a 2D testcase (upper figure) and a 3D testcase (lower figure);

Fig. 5: an exemplary mixing reactor;

Fig. 6: an exemplary computational grid for the mixing reactor of fig. 5;

Fig. 7: an exemplary modified computation grid of fig. 6; and

Fig. 8: another exemplary modified computation grid of fig. 6.

## Detailed Description

The method according to the invention may comprise and/or involve a Lattice Boltzmann Method (LBM). The collision step and/or the streaming step may comprise, involve and/or be part of a Lattice Boltzmann Method (LBM). The method according to the invention may comprise and/or involve solving a Lattice Boltzmann equation.
The Lattice Boltzmann Method (LBM) can be a discretization on a lattice and/or computational grid of the basic kinetic Boltzmann equation. The lattice and/or computational grid may be uniform. The lattice may comprise, be and/or correspond to the computational grid.
By employing the Lattice Boltzmann Method, and/or a Lattice Boltzmann equation (LBE), the distribution function may be computed at grid points of the computational grid.
The distribution function may be a distribution function of a velocity component of a flow in some direction, e.g. in a lattice direction (and/or in a direction of the computation grid, and/or along an axis). In some embodiments, a distribution function, and/or the distribution function, may be a distribution function of a species.
The distribution function may be a probability density function. The distribution function may be a probability density function of having a flow velocity (e.g. in lattice direction or the like) at a grid point, and/or of finding a fluid particle at a grid point. The distribution function may be a probability density function of having a concentration of a species $\alpha$ at a grid point.
In some embodiments, it may be provided that more than one distribution function may be computed and/or

solved.

In figure 1, an exemplary computational grid (and/or lattice) is shown. The distribution function, the flow properties, flow velocities and/or the like may be discretized and/or computed at grid points of the computational grid. The discretization may be a D2Q9 discretization. The computational grid may be 2D (two-dimensional). The discretization, e.g. of velocity space and/or species, may be into nine directions (cf. figure 1).

Other discretizations are possible. For instance, the computational grid may be 3D (three-dimensional). The discretization may be a D3Q15 discretization. The discretization may be a D3Q27 discretization.

In case of absence of external forces and constant temperature, a distribution function according to LBM can be given and/or computed by

$$f_k^\alpha\left(\mathbf{x}+\mathbf{e}_k\Delta t, t+\Delta t\right)-f_k^\alpha\left(\mathbf{x}, t\right)=-\frac{\Delta t}{\tau}\left(f_k^\alpha\left(\mathbf{x}, t\right)-f_k^{eq,\alpha}\left(\mathbf{x}, t\right)\right),$$

with k as index of the direction (e.g. k = 0, 1, ..., 8 in case of D2Q9, cf. e.g. fig. 1), $\tau$ a nondimensional relaxation time, x a position in space, $e_k$ a lattice velocity, $\alpha$ a species and $\Delta t$ a time step. In other words, it may be provided that the evolution of the distribution function as function of space and time may be given by the above equation. Here, $f^{eq,\alpha}$ is an equilibrium distribution function of species a, which may depend on the position and time.

The above equation can be solved in two steps. In a first step, which can be, comprise or correspond to the collision step, an intermediate distribution function may be computed by

$$\widetilde{f_k^\alpha}\left(\mathbf{x}, t\right)=-\frac{\Delta t}{\tau}\left(f_k^\alpha\left(\mathbf{x}, t\right)-f_k^{eq,\alpha}\left(\mathbf{x}, t\right)\right)$$

The intermediate distribution function may be based on the difference between the distribution function and the equilibrium distribution function.

Then, in a second step, which can be comprise or correspond to the streaming step, the distribution function at time step t + $\Delta$t and position **x** + $\mathbf{e}_k$ $\Delta$t can be determined as

$$f_k^\alpha\left(\mathbf{x}+\mathbf{e}_k\Delta t, t+\Delta t\right)=\widetilde{f_k^\alpha}\left(\mathbf{x}, t\right).$$

This is shown for illustrative purposes in fig. 1 (right), where a fluid particle 1 is moved as determined in the streaming step. The fluid particle 1 may be move from a grid point 30 to an adjacent and/or neighboring grid point 30. Alternatively, the fluid particle 1 may stay at its current grid point 30.

In case of D2Q9, the lattice velocity $\mathbf{e}_k$ can be given by e.g.

$$\mathbf{e}_k=\begin{pmatrix}0 & 1 & 0 & -1 & 0 & 1 & -1 & -1 & 1\\ 0 & 0 & 1 & 0 & -1 & 1 & 1 & -1 & -1\end{pmatrix}\frac{\Delta x}{\Delta t},$$

cf. Figure 1. For different discretizations, such as e.g. D3Q15 or D3Q27, $e_k$ would be chosen differently. In other words, the lattice velocity can depend on the ratio of the distance between grid points $\Delta$x and the time step $\Delta$t, and/or can be such that in a time increment $\Delta$t, the distance $\Delta$x between neighboring grid points can be traversed.

Without loss of generality, in some embodiments $\Delta$x = 1 and $\Delta$t = 1. In some embodiments, a suitable normalization and/or non-dimensionalization may be used.

The notation of the distribution function $f_k^\alpha(x,t)$ may be equivalent to f($x_1$, $x_2$, $v_1$, $v_2$, a) for the two-dimensional case, and equivalent to f(xi, $x_2$, $x_3$, $v_1$, $v_2$, $v_3$, a) for the three-dimensional case. It may be provided that the distribution function, or respectively the corresponding tensor, does not have a specific index for the time t. In some embodiments, it may be provided that the distribution function, and/or the corresponding tensor, is updated and/or overwritten at each time step.

Alternatively or additionally, the notation of the distribution function $f_k^\alpha(x,t)$ may be equivalent to f(xi, $x_2$, $v_1$,

$v_2$, t, a) for the two-dimensional case, and equivalent to f(xi, $x_2$, $x_3$, $v_1$, $v_2$, $v_3$, t, a) for the three-dimensional case. It may be provided that the distribution function, or respectively the corresponding tensor, has an index for the time t. In some embodiments, it may be provided that the distribution function, and/or the corresponding tensor, contains a collection of values for at least two and/or all time steps, e.g. distinct time steps of the simulation. The at least two time steps can be different and/or distinct time steps. In some embodiments, at least two, multiple and/or all time steps of the tensor are time steps immediately computed one after the other, and/or may be separated by Δt. Alternatively or additionally, at least two, multiple and/or all time steps of the tensor may be separated by one or more intermediate time step or time interval, e.g. one or more intermediate time steps of the simulation, and/or separated by more than Δt.

The indices $x_1$, $x_2$ may correspond to the position of grid points in case of a two-dimensional grid or lattice. The indices $x_1$, $x_2$, $x_3$ may correspond to the position of grid points in case of a three-dimensional grid or lattice. The index $x_1$ may correspond to a direction and/or index x and/or be also called x, the index $x_2$ may correspond to a direction and/or index y and/or be also called y, and/or the index $x_3$ may correspond to a direction and/or index z and/or be also called z.

The size of the index $x_1$ may be equal to the number of grid points along an $x_1$-axis (or x-axis) of the computational grid. The size of the index $x_2$ may be equal to the number of grid points along an $x_2$-axis (or y-axis) of the computational grid. The size of the index $x_3$ may be equal to the number of grid points along an $x_3$-axis (or z-axis) of the computational grid.

The number of grid points along an axis j may be $n_j$. The index j may correspond to a direction in space, and may be e.g. j = 1, 2 for the two-dimensional case and j = 1, 2, 3 for the three-dimensional case. In some embodiments, the number of grid points along at least two axis, and/or along all axis may be equal, such that $n_j$ = n for all j. In some embodiments, the computational grid and/or lattice may have $n_j = 2^m$ grid points for at least some axis j. In some embodiments, the computational grid and/or lattice may have $n_j = 3^m$ grid points for at least some axis j. The total number of grid points may be equal to $n_1n_2$ for the two-dimensional case, and/or may be equal to $n_1n_2n_3$ for the two-dimensional case. In some embodiments, the total number of grid points maybe $(2^m)^j$. In some embodiments, the total number of grid points maybe $(3^m)^j$.

The indices $v_1$, $v_2$ may correspond to the lattice velocity components in case of a two-dimensional grid or lattice. The indices $v_1$, $v_2$, $v_3$ may correspond to the lattice velocity in case of a three-dimensional grid or lattice. The index $v_1$ may correspond to a lattice velocity and/or index $v_x$ and/or be also called $v_x$, the index $v_2$ may correspond to a direction and/or index $v_y$ and/or be also called $v_y$, and/or the index $v_3$ may correspond to a direction and/or index $v_z$ and/or be also called $v_z$.

The lattice velocity components may have values equal to -Δx / Δt, 0, or Δx / Δt (cf. for the case D2Q9 with the lattice velocity $e_k$ given above). Here, Δx can be the distance between grid points and Δt the time step, so that c = Δx / Δt is a lattice speed.

The nondimensional relaxation time τ can be related to the kinematic viscosity by

$$\nu = (\tau - 0.5)c_s^2.$$

The speed of sound $c_s$ may be given by $c_s = c/\sqrt{3}$, wherein c is a lattice speed, c = *Δx*/ *Δt* with Δx the distance between grid points, and Δt the time step and/or increment between adjacent timesteps.

For solving the equation above, an equilibrium solution is required. In some embodiments, the equilibrium solution may be or given and/or computed by

$$f_k^{eq,\alpha} = w_k \rho^\alpha \left[1 + \frac{3}{c^2}\mathbf{e}_k \cdot \mathbf{u} + \frac{9}{2c^4}(\mathbf{e}_k \cdot \mathbf{u})^2 + \frac{3}{2c^2}\mathbf{u} \cdot \mathbf{u}\right]$$

where $\rho^\alpha$ is a fluid density of species α, **u** is a macroscopic velocity and c the lattice speed. $w_k$ may be weighting coefficients, which may depend on the direction k. For instance, with reference to fig. 1 (left), the weighting coefficients may be given by $w_0$ = 4/9, $w_1 = w_2 = w_3 = w_4$ = 1/9, and $w_5 = w_6 = w_7 = w_8$ = 1/36. In some embodiments, the weighting coefficients may be different. The weighting coefficients may depend on the discretization scheme DxQy. The weighting coefficients may be chosen such that they sum to unity, $\Sigma_k w_k = 1$.

The fluid density of species α may be defined and/or computed as

$$\rho^\alpha(\mathbf{x}, t) = \sum_k f_k(\mathbf{x}, t) .$$

The macroscopic velocity may be given by

$$\mathbf{u}(\mathbf{x},t) = \frac{\sum_\alpha \rho^\alpha(\mathbf{x},t)\,\mathbf{u}^\alpha(\mathbf{x},t)}{\sum_\alpha \rho^\alpha(\mathbf{x},t)},$$

i.e. may correspond to the density weighted macroscopic velocity of species a. Herein,

$$\rho^\alpha(\mathbf{x},t)\,\mathbf{u}^\alpha(\mathbf{x},t) = \sum_k \mathbf{e}_k f_k^\alpha(\mathbf{x},t).$$

The invention is not limited to the above equations. Depending on the purpose, external effects and/or external forces, temperature effects, or the like, and/or other equations and/or models may be employed.

Figures 2 and 3 show exemplary embodiments of tensor-train decompositions.

Figure 2 shows a schematic decomposition of a tensor A(x, y, z) into a tensor train (TT), cf. the upper part of figure 2 (where the tensor A is indicated with reference numeral 2). Figure 2 further shows a decomposition in quantized tensor train format (QTT), cf. the lower part of figure 2. The distribution function may be or comprise a tensor 2.

A may be a function depending on x, y and z. Alternatively or additionally, A may be some quantity, e.g. a temperature, density or the like, which may e.g. vary in the tree-dimensional space as spanned by x, y and z. The quantity may be given at grid points at positions x, y and z. The tensor A may have n grid points in each direction x, y, and z, and/or the size of the indices x, y and z may be n. The tensor A may be a three-dimensional tensor. This is indicated by the three legs x, y and z in the upper left of fig. 2, where n indicates respectively the number of entries of each index x, y and z.

In general, the tensor train format (TT format) may correspond to a decomposition of the (general) tensor A (cf. e.g. Oseledets and Tyrtyshnikov, "Tt-cross approximation for multidimensional arrays", Linear Algebra and its Applications, 432(1):70-88, 2010) as given by

$$A(i_1,\ldots,i_d) = \sum_{\beta_0,\ldots,\beta_{d-1},\beta_d} G_1(\beta_0,i_1,\beta_1)G_2(\beta_0 1,i_2,\beta_2)\ldots G_d(\beta_{d-1},i_d,\beta_d)$$

where $G_j$ may be tensor train cores 5 (TT-cores 5), where $G_j$ and/or the tensor train core 5 is or may be a 3-dimensional tensor. The main characteristic of such a representation is the rank r, which is equal to the maximum size among the indexes $\beta_0$, $\beta_1$, ..., $\beta_d$. and which expresses the correlations and entanglement in the tensor. In case of weak correlations (low rank r), this format allows to store and perform operations with tensors with logarithmic complexity in its size.

Mainly, in the present invention d-dimensional tensors A are employed. In some embodiments, each index of tensor A can have n different values, so that there are $n^d$ elements in tensor A which usually results in the same complexity of operations. Importantly, any tensor can be represented as a tensor train of rank r via singular value decomposition (SVD) (cf. e.g. Melven, Thies, and Basermann, Performance of the low-rank tt-svd for large dense tensors on modern multicore cpus, SIAM Journal on Scientific Computing, 44(4):C287-C309, 2022) or cross-approximation (cf. e.g. Oseledets and Tyrtyshnikov, "Tt-cross approximation for multidimensional arrays", Linear Algebra and its Applications, 432(1):70-88, 2010). Such a representation possesses only $dnr^2$ elements. If the rank is restricted, which is indeed the case, e.g. in PDE solutions (cf. e.g. Dolgov, Khoromskij, and Oseledets, "Fast solution of parabolic problems in the tensor train/quantized tensor train format with initial application to the fokker-planck equation", SIAM Journal on Scientific Computing, 34(6):A3016-A3038, 2012 or Gourianov, Lubasch, Dolgov, van den Berg, Babaee, Givi, Kiffner, and Jaksch, "A quantum-inspired approach to exploit turbulence structures", Nature Computational Science, 2(1):30-37, 2022) and representations of some smooth multi-dimensional functions (cf. e.g. Rohrbach, Dolgov, Grasedyck, and Scheichl, "Rank bounds for approximating gaussian densities in the tensor-train format", arXiυ preprint arX1υ:2001.08187, 2020), then an exponential compression is obtained. Besides, almost all algebraic operations in the TT format have a similar complexity (cf. Oseledets, "Tensor-train decomposition", SIAM J. Scientific Computing, 33:2295-2317, 01 2011) as shown in the following table.

| Number | Operation | Result rank | Complexity |
|---|---|---|---|
| 1 | $\mathbf{C} = \mathbf{A} \cdot \text{const}$ | $r(\mathbf{C}) = r(\mathbf{A})$ | $O(dr(\mathbf{A}))$ |
| 2 | $\mathbf{C} = \mathbf{A} + \mathbf{B}$ | $r(\mathbf{C}) \leq r(\mathbf{A}) + r(\mathbf{B})$ | $O(nd(r(\mathbf{A}) + r(\mathbf{B}))^2)$ |
| 3 | $\mathbf{C} = \mathbf{A} \odot \mathbf{B}$ | $r(\mathbf{C}) \leq r(\mathbf{A})r(\mathbf{B})$ | $O(ndr^2(\mathbf{A})r^2(\mathbf{B}))$ |
| 4 | sum **A** | - | $O(ndr^2(\mathbf{A}))$ |
| 5 | **A[i]** | - | $O(ndr^2(\mathbf{A}))$ |

(continued)

| Number | Operation | Result rank | Complexity |
|---|---|---|---|
| 6 | **C** = round(**A**, $\varepsilon$) | r(**C**) < r(**A**) | $O$ ($ndr^3$(**A**)) |

It can be seen that with a small rank value, all these operations are much more efficient to perform in TT format. Another important TT-algorithm, which may be used in some embodiments, is the cross-approximation technique (cf. Oseledets and Tyrtyshnikov, "Tt-cross approximation for multidimensional arrays", Linear Algebra and its Applications, 432(1):70-88, 2010). This algorithm allows to recover a tensor train of rank r from a given tensor referring only to $O(dnr^2)$ of its elements. In the context of this invention, this means that we can apply almost any function to a tensor train for $O(d^2n^2r^4)$ operations, because the access to the tensor train element also has complexity $O(dnr^2)$, cf. operation 5 in the table above. The overall scaling can be enhanced to $O(dnr^2)$ if parallelization is used - each iteration of the cross-approximation requires $nr^2$ calls to the tensor train and they can be performed in parallel; also passing through d tt-cores when calling a tensor train can be parallelized.

Since in physical problems the dimension of space is usually no higher than 3 (d = 3), then for more compression, one can use the so-called Quantized Tensor Train (QTT) format. It is obtained by introducing artificial indexes, i.e. by "reshaping" of visible indexes into twos or threes, as shown in Fig. 2. (lower part) and Fig. 3. For instance, if the size of an index, e.g. index $x_1$, is equal to $2^m$, then one might reshape the index into a tensor

$$\underbrace{\mathbb{R}^2 \otimes \cdots \otimes \mathbb{R}^2}_{\text{m times}}.$$

Similarly, if the size of an index, e.g. index $x_1$, is equal to $3^m$, then one might reshape the index into a tensor

$$\underbrace{\mathbb{R}^3 \otimes \cdots \otimes \mathbb{R}^3}_{\text{m times}}.$$

It may be provided that the tensor train cores 5 are further reshaped and/or "refined" into quantized tensor train cores, as e.g. shown in fig. 2 (lower part).

For the QTT format, all the stated above also holds true as for the standard tensor train format.

Figure 3 shows exemplary distribution functions f in QTT format, where the index $\alpha$ has been omitted. In some embodiments, the distribution functions f in fig. 3 can be distribution functions of a single species. The exemplary distribution functions of fig. 3 (in the upper part of the figure) may be distribution functions in a D2Q9 case, where the grid size is equal to $3^d$ per axis. The exemplary distribution functions of fig. 3 (in the lower part of the figure) may be distribution functions in a D2Q9 case, where the grid size is equal to $2^d$ per axis. Further, the exemplary distribution function as shown in the lower part of the figure may explicitly comprise an index t with size $2^s$, which explicitly corresponds to different timesteps. The distribution function f may be or comprise a tensor 2.

For the shown exemplary distribution functions of fig. 3, the velocities $v_x$, $v_y$ have only 3 values per axis (cf. the D2Q9 case of fig. 1), i.e. may have values of - $\Delta x$ / $\Delta t$, 0 and/or $\Delta x$ / $\Delta t$. Without loss of generality, the velocities may have values of -1, 0 and/or 1 in some embodiments.

All steps of the method according to the invention can be carried out with the distribution function in TT format and/or QTT format. All operations can be carried out without leaving the TT format and/or the QTT format. Thereby, a significant speed-up can be achieved as compared to methods known from the art. Further, the method according to the invention may require much less memory than methods known from the art. For a given amount of memory, such as e.g. RAM of a computer system and/or a given number of computer nodes in a distributed computing system, a much finer computational grid 20 can be used when using the method according to the invention as compared to the art.

For instance in some embodiments, for the collision step, firstly the macroscopic velocity u is calculated. Multiplication by $e_k$ and summation are done straightforward (operations 3 and 4 operations in the above table). To divide by density $\rho$, the cross-approximation can be used. When the velocity is calculated, all other operations are just multiplication by a constant, element-wise multiplication and summation (operations 1 - 3 in the above table). At the end, the inflated ranks can be truncated using the rounding operation 6 of the table above.

In some embodiments, for the streaming step, which consists of the application of a shift function over the tensor train, the cross-approximation algorithm can be used.

Thus, it is possible to implement all LBM steps using tensor trains. The algorithm based on tensor trains provides O(d) runtime scaling, while the classical implementation has a complexity of $O(3^{2d})$.

In figure 4, runtime comparisons for some methods according to the invention and methods known from prior art

are shown. The respective methods differ in that the methods according to the invention, a QTT decomposition of the distribution function was employed, wherein the methods known from prior art did not use tensor-train decomposition. Specifically, LBM with QTT decomposition according to an embodiment of the invention, and classical LBM as known from the art was compared for the Taylor-Green Vortex in 2D and 3D. It is well known that the Taylor-Green Vortex has an analytical solution, which may be used to compare the accuracy of different methods.

The spatial domain of an exemplary test case is a square $[0, 2\pi] \times [0, 2\pi]$ with periodic boundary conditions for the 2D case. For the 3D case, the spatial domain of an exemplary test case corresponds to a cube with edge length $L = 2\pi$ (i.e. a cube $[0, 2\pi]^3$) with periodic boundary conditions.

Noticeably, for the 2D case, 12 GB of memory is not enough to store the entire tensor $f(x, y, v_x, v_y)$ if $3^9 * 3^9 = 19683 * 19683$ grid points are used to discretize the spatial domain. If one chooses double precision, then $19683^2 * 3^2 * 8$ byte ~ 25,97 Gb memory is required.

For the 2D test case, the flow of a single species was simulated each with QTT LBM and classical LBM, with density $\rho = 1$, sound of speed $c = 1$, initial speed $u_0 = 0.01$ (thus Mach number Ma = 0.01), the initial distribution function $f(t = 0) = f^{eq}$ and Re = 1. The resulting runtime as function of the number of grid points is shown in figure 4 (upper part), where the QTT LBM corresponds to the solid line and the classical LBM to the dashed line.

Clearly, there is a significant speedup of the method according to the invention over the prior art, when the computational grid is fine enough.

For the 3D testcase, a small number of Re = 1, Mach number Ma = 0.01, speed of sound c = 1, size of the area $L = 2\pi$, and number of points along each of the axes $N = 8_1$ (i.e. $N = 3^4$) was chosen. In the lower figure of fig. 4, the runtime is plotted as function of the time step $\Delta t$ for the 3D testcase, where the characteristic time corresponds to suitably normalized and non-dimensional time step $\Delta t$. The error of QTT LBM to the analytical solution was smaller than $7 * 10^{-5}$. Clearly, the method according to the invention solves the problem faster than the method from the art.

Since the Courant-Friedrichs-Lewy (CFL) number for LBM has to be 1, because the particle distribution function can only be shifted between neighboring lattice points in a single time-step, the time step may be small when the computational grid is fine (i.e. small $\Delta x$), $\Delta t = \Delta x$. Thus, a large number of iterations may be required.

However, the methods according to the invention also allow to solve the Lattice Boltzmann equation (LBE) in TT format and/or QTT format to overcome this issue. In some embodiments, the Lattice Boltzmann equation may be given by

$$\frac{\partial f_k}{\partial t}(\mathrm{x}, t) + \mathrm{e}_k \cdot \nabla f_k(\mathrm{x}, t) = -\frac{1}{\tau}\left(f_k(\mathbf{x}, t) - f_k^{eq}(\mathbf{x}, t)\right) .$$

Using an implicit scheme may yield

$$\frac{f^{i+1} - f^i}{\Delta t} + \mathbf{e} \cdot \nabla f^{i+1} = -\frac{1}{\tau}\left(f^{i+1} - f^{eq}(f^i)\right),$$

where e.g. $f^i = f(x, y, v_x, v_y)$ in 2D and/or e.g. $f^i = f(x, y, z, v_x, v_y, v_z)$ in 3D may be a distribution function at time step i. The explicit reference to species $\alpha$ is omitted in the following. However, the method is also suitable for computing mixtures of at least two species a, where the equations can be adapted as necessary, as is obvious to the person skilled in the art. Thus, the linear system of equations

$$\underbrace{\left(\left(\frac{1}{\Delta t} + \frac{1}{\tau}\right) I + \mathbf{e} \cdot \nabla\right)}_{A} f^{i+1} = \underbrace{\frac{f^i}{\Delta t} + \frac{f^{eq}(f^i)}{\tau}}_{b}$$

may be solved, where the r.h.s. b may correspond to the collision step and the l.h.s A to the streaming step. This amounts to solving a linear system $A\,x = b$, where both A and b can be represented in TT format and/or QTT format. Thus, efficient methods for linear systems solutions can be used, such as the Alternating Minimal Energy (AMEn) approach (cf. Dolgov and Savostyanov, "Alternating minimal energy methods for linear systems in higher dimensions", SIAM Journal on Scientific Computing, 36(5):1-24, September 2014), which has a complexity $O(dr^b)$.

In another way to solve LBE using QTT according to the invention, the entire distribution function $f = f(x, y, v_x, v_y, t)$ and/or $f = f(x, y, z, v_x, v_y, v_z, t)$ may be represented in a quantized tensor train format. The explicit reference to species $\alpha$ is omitted in the following. However, the method is also suitable for computing mixtures of at least two species a, where the equations can be adapted as necessary, as is obvious to the person skilled in the art.

In other words. The distribution function f is in a quantized tensor train format both in space and time, cf. e.g. the lower part of figure 3.

Then, the solution of the LBE can be reduced to the solution of the following nonlinear equation

$$\underbrace{\left(\frac{1}{\Delta t}A_t + \frac{1}{\Delta x}A_{\mathbf{x}} + \frac{1}{\tau}I\right)}_{A} f = \frac{1}{\tau}f^{eq}(f) + \frac{1}{\Delta t}f(t=0) \otimes e_1$$

,

where corresponding matrices can be written down through the tensor products $A_t = I_x \otimes I_y \otimes I_{v_x} \otimes I_{v_y} \otimes \Delta_1^t$, $A_{\mathbf{x}} = \Delta_2^x \otimes I_y \otimes \hat{v}_x \otimes I_{v_y} \otimes I_t + I_x \otimes \Delta_2^y \otimes I_{v_x} \otimes \hat{v}_y \otimes I_t$. Here, I is the identity matrix and ei = (1, 0, ..., 0)$^T$ is the first unit vector. Further, the velocities matrix $\hat{v}_x = \hat{v}_y = diag(0, 1, -1)$. $\Delta_1$ is a matrix of the first derivative of the direct order

$$\Delta_1 = \begin{pmatrix} 1 & 0 & & & \\ -1 & 1 & 0 & & \\ & \ddots & \ddots & \ddots & \\ & & -1 & 1 & 0 \\ & & & -1 & 1 \end{pmatrix}.$$

In case of periodic boundary conditions,

$$\Delta_2 = \frac{1}{2}\begin{pmatrix} -1 & 0 & \cdots & 0 & 1 \\ -1 & 1 & \cdots & 0 & 0 \\ 0 & \ddots & \ddots & \ddots & \\ & & & & 0 \\ 0 & \cdots & 0 & -1 & 1 \\ 1 & 0 & \cdots & 0 & -1 \end{pmatrix}.$$

In case of different boundary conditions, adaptations can be made which are obvious to the person skilled in the art.

Both $A_t$ and $A_x$ can be well represented in QTT format with rank 3. To solve this nonlinear equation, e.g. the Picard iteration method can be used,

$$Af^{i+1} = \frac{1}{\tau}f^{eq}(f^i) + \frac{1}{\Delta t}f(t=0) \otimes e_1.$$

To solve this equation at each step, efficient tensor-train methods can be used, such as e.g. the AMEn algorithm mentioned above.

Figures 5 shows an exemplary embodiment of a mixing reactor 10. In some embodiments, the mixing reactor 10 may be or comprise a T-mixing reactor. The mixing reactor 10, and/or at least a mixing chamber of the mixing reactor, may be T-shaped.

Alternatively or additionally, in some embodiments the mixing reactor 10 may be or comprise a batch reactor, and/or a flow reactor.

In the T-shaped reactor of fig. 5, fluids (e.g. of different species) may flow into the reactor at the arms of the T-shape (left side of figure 5). The fluids may mix in the reactor, and the mixture may flow out at the long end of the T-shape (right side of figure 5).

For instance, a first fluid may enter the mixing reactor 10 via an inlet (cf. inflow 70). A second fluid may enter the mixing reactor 10 via an second inlet (cf. inflow 80). The first fluid may comprise or consist of a first species a, or comprise or consist of at least one species a. The second fluid may comprise or consist of a second species a. In some embodiments, the first species and the second species may be different. In some embodiments, the first species and the second species

may be the same. Alternatively or additionally, the first fluid comprises at least one species, which may be different from at least one, multiple or all species of the second fluid. Alternatively or additionally, at least one, multiple or all species of the first fluid may be the same as at least one, multiple or all species of the second fluid. In some embodiments, the first fluid and/or the second fluid may comprise or consist of a mixture of different species.

In some embodiments, the first fluid and/or the second fluid may have the same temperature. Alternatively, it may be provided that the temperature of the first fluid is different from the temperature of the second fluid, e.g. when entering the mixing reactor 10.

The first fluid and/or inflow 70 may enter the mixing reactor 10 with a specific inflow velocity. It may be provided that a specific mass flow rate and/or volume flow rate of first fluid and/or inflow 70 enters the mixing reactor 10. The second fluid and/or inflow 80 may enter the mixing reactor 10 with a specific inflow velocity. It may be provided that a specific mass flow rate and/or volume flow rate of second fluid and/or inflow 80 enters the mixing reactor 10.

An inlet for the first fluid and/or inflow 70 may have an inflow cross-section $A_i$. An inlet for the second fluid and/or inflow 80 may have an inflow cross-section $A_I$, which may be equal or different to the cross-section of the inlet for the first fluid and/or inflow 70.

The first fluid and/or inflow 70 may be or comprise a liquid, a gas, a multi-phase flow, or the like. The second fluid and/or inflow 80 may be or comprise a liquid, a gas, a multi-phase flow, or the like.

In the mixing reactor 10, the first fluid and the second fluid may mix. In some embodiments, the first fluid and the second fluid may react to at least one reaction product. In some embodiments, when mixing, at least one or multiple of the density, temperature, composition, concentration, species, pressure, or the like may change. In some embodiments, when mixing, the first fluid and the second fluid may react.

It may be provided that the mixture has a density, temperature, composition, concentration, species, pressure, or the like which may be different from first fluid and/or the second fluid.

The mixture may leave the mixing reactor 10. An outflow 80 of the mixing reactor 10 may be or comprise the mixture. In some embodiments, the outflow 80 of the mixing reactor 10 may comprise some of first fluid and/or second fluid.

The outflow 80 may leave the mixing reactor 10 via an outlet. The outlet may have an outlet cross-section $A_O$. The outflow 80 may leave the mixing reactor 10 with a specific outflow velocity. It may be provided that a specific mass flow rate and/or volume flow rate of the outflow 80 leaves the mixing reactor 10.

The mixing reactor 10 may have a characteristic dimension. In some embodiments, the characteristic dimension may be a mixing length L, e.g. when the mixing reactor 10 is T-shaped. The mixing length L may be e.g. a length along which mixing of inflows 70 and 80 may take place.

The mixing reactor 10 may have one or more sensors 100. The sensor 100 may be configured to measure or determine a property of a fluid, e.g. at and/or near a measuring point. The measuring point may be in the mixing reactor 10, and/or at a wall of mixing reactor 10. The property may be or comprise one or more of fluid velocity, density, temperature, pressure, concentration and/or composition. The property may be or comprise a species concentration, such as e.g. a species concentration of the fluid and/or mixture in the mixing reactor 10. The property may be or comprise a composition of the fluid and/or mixture in the mixing reactor 10.

The mixing reactor 10 may have a temperature control unit 110. The temperature control unit 110 may be configured to heat and/or cool walls of the mixing reactor 10, and/or to impose a temperature profile and/or temperature distribution.

In some embodiments, the mixing reactor 10 may be or comprise a batch reactor (not shown in the figures). In some embodiments, the mixing reactor 10 may be or comprise a continuous reactor (not shown in the figures). It may be provided that the mixing reactor 10 has at least one agitator (not shown in the figures). The agitator may facilitate mixing in the mixing reactor. In some embodiments, the agitator may be configured to stir or whirl fluid in the mixing reactor 10.

A control unit may be provided (not shown in the figures). The control unit may be part of the mixing reactor 10, or separate from it. The control unit may be configured to control the mixing reactor 10, and/or the mixing in the mixing reactor 10. In some embodiments, the control unit may be configured to carry out one, multiple or all methods of the invention.

The mixing reactor 10 may have more than one, and/or more than two, inflows and/or outflows. The mixing reactor 10 may have a geometry different from a T-shape. The mixing reactor 10 may be configured to mix more than two inflows, and/or educts. The mixing reactor 10 may be configured to provide more than one mixture or product.

The method for simulating fluid flow is not limited to simulate flow in a mixture reactor 10. In some embodiments, the method for simulating fluid flow can be used to simulate fluid flow in, around and/or through arbitrary shapes, forms, vessels, conduits, pipes, chambers, reactors, devices or the like.

An embodiment of a method according to the invention is used to simulate a fluid flow. The method can be used to determine a distribution function of the fluid flow. Alternatively or additionally, the method can be used to determine a distribution function of a mixture of at least two species. The method may be computer-implemented.

An embodiment of a method according to the invention is used to design a mixing reactor 10, and/or when designing a mixing reactor. The method may be computer-implemented.

The method for designing a mixing reactor 10 comprises providing an initial mixing reactor geometry. The mixing

reactor geometry may comprise or consist of a computational grid 20 with grid points 30. Alternatively or additionally, a computation grid and/or grid points may be created based on the initial mixing reactor geometry. The mixing reactor geometry. An exemplary initial mixing reactor geometry for the mixing reactor 10 of fig. 5 is shown in fig. 6.

The number of grid points 30, and/or the distance $\Delta x$ between adjacent grid points 30, may be exemplary. In some embodiments, a different number of grid points 30, and/or a distance $\Delta x$, can be chosen or provided.

The computational grid 20 may be uniform and/or rectangular. In some embodiments, the computational grid 20 may be non-uniform and/or non-rectangular. It may be provided that the mesh distance may be different for different axis, and/or vary at least locally.

The computational grid 30 and/or the simulation can use and/or have suitable boundary conditions. For instance, wall boundary conditions (e.g. von Neumann and/or Dirichlet boundary conditions) may be employed to represent (physical) walls of the mixing reactor 10. Inlets and/or outlets, and/or inflows and/or outflows, can be modeled and/or taken into account by suitable boundary conditions, e.g. determined or given profiles and/or values of velocity, pressure, density, temperature or the like. Inflows and/or outflows can be modeled and/or taken into account by suitable boundary conditions representing (physical) inflows into, and/or outflows out of, mixing reactor 10.

In some embodiments, the temperature control unit 110 can be modeled and/or taken into account by e.g. determined or given profiles and/or values of the temperature at the boundaries.

In some embodiments, agitators or the like may be taken into account, when the mixing reactor to be designed has at least one agitator.

The method for designing a mixing reactor 10 comprises a step of simulating a mixing of at least two species, and/or a flow in and/or through the mixing reactor, via a method for simulating fluid flow according to the invention. A characteristic property of the mixture and/or the fluid flow can be determined when or after carrying out the simulation. In some embodiments, the characteristic property can be or comprise at least one of a mixing fraction, a species concentration, a characteristic mixing time, a characteristic residence time and/or a mixing progress. The characteristic property maybe or comprise one or more statistical quantities, such as e.g. an average, variance, or higher moment. It may be provided that the statistical quantity may be an ensemble average, a time average, and/or a density-weighted average, and/or a statistical quantity evaluated and/or computed in space and/or time. In some embodiments, the characteristic property may be computed at or for at least one, multiple or all grid points. The characteristic property may be or comprise a scalar quantity. The characteristic property may be or comprise a directional quantity, such as e.g. a vector or a tensor of higher order.

The method for designing a mixing reactor 10 further comprises a step of modifying the mixing reactor geometry and/or grid points 30. When or after modifying the mixing reactor geometry and/or grid points 30, the simulation may be repeated using the modified mixing reactor geometry and/or grid points 30. When or after repeating the simulation, the characteristic property of the mixture can be determined.

The characteristic property of consecutive repeats of the simulation can be compared. In some embodiments, the characteristic property of at least two, multiple or all simulation runs with different reactor geometry can be compared. When comparing the characteristic property, the characteristic property can be compared to a target value of the characteristic property.

By repeating and/or iterating the steps, the mixing reactor geometry can be optimized. The iteration can end when the characteristic property is close enough to the target value. The iteration can end when the characteristic property has an accuracy close enough to the target value and/or deviates less than a given value from the target value.

In some embodiments, when optimizing the mixing reactor geometry, more than one characteristic property can be taken into account. The mixing reactor geometry can be optimized with respect to more than one characteristic property.

Modifying the mixing reactor geometry can comprise moving a boundary and/or changing the shape of the computational grid 20. Alternatively or additionally, modifying the mixing reactor geometry can comprise adding or removing grid 30 points from the computational grid 20, and/or coarsening and/or refining the computational grid 30.

For instance, fig. 7 shows an exemplary embodiment where the grid points 30 of computational grid 20 of fig. 6 have been modified. The grid spacing (in x and/or y-direction in fig 7) may have been adjusted and/or changed, in some embodiments locally. Additionally or alternatively, the computational grid 20 has been coarsened. Grid points 30 have been removed from the computational grid 20. The embodiment as shown in fig. 7 is only exemplary.

Fig. 8 shows an exemplary embodiment where the mixing reactor geometry of fig. 6 has been modified. Particularly, the dimensions of the mixing reactor have been changed such that the characteristic property is optimized, close enough to a target value and/or within a given interval. For instance, the characteristic dimension may have been changed. In the exemplary embodiment of fig. 8, the mixing length L, inlets, e.g. the cross-section $A_I$, and/or outlet, e.g. cross-section $A_O$, may be changed compared to the initial mixing reactor geometry. The mixing length L may be shorter than for the initial mixing reactor geometry (cf. with fig. 6), one of the inlets may have a larger cross-section $A_I$, the outlet may have a larger cross-section $A_O$, and/or the length of one "arm" of the T-shape (e.g. extending from the upper inlet) may be increased. The embodiment as shown in fig. 8 is only exemplary.

After the mixing reactor geometry has been optimized, the resulting computational grid 30 can be used as design

for a (physical) mixing reactor 10. The design, and/or technical drawings, may be based on and/or construed from the resulting mixing reactor geometry.

Thus, a mixing reactor 10 (e.g. as shown in fig. 5) for mixing at least two species may be designed based on and/or using the method for designing a mixing reactor according to the invention.

The inventive method for designing a mixing reactor may be faster and requires less memory than methods based on the known art, particularly for complex geometries. For instance, complex geometries require fine computational grids with many grid points, which in turn requires a lot of memory for computation. Thereby, the method for designing a mixing reactor according to the invention allows for quick and accurate design of mixing reactors.

The invention further pertains to a mixing reactor 10 (as e.g. shown in fig. 5) which has a mixing reactor geometry as obtained by the method for designing a mixing reactor according to the invention. The geometry may be an inner geometry of the mixing reactor 10.

The invention further relates to a method for controlling a mixing reactor 10, e.g. a mixing reactor 10 shown in fig. 5.

The method for controlling the mixing reactor 10 comprises a step of simulating a mixing of at least two species, and/or a flow in and/or through the mixing reactor, via the method of simulating a fluid flow according to the invention. The computational grid corresponds to a geometry of the mixing reactor 10.

The method for controlling the mixing reactor 10 further comprises a step of generating and/or updating at least one control command for the mixing reactor 10 based on the simulation.

The method for controlling the mixing reactor can be computer-implemented.

Updating the control command can comprise adapting, modifying and/or changing a control command, such as e.g. an existing and/or previously generated control command.

The control command can comprise one or more of a command for controlling an inflow of at least one flow into the mixing reactor and/or an outflow of at least one flow out of the reactor. The control command can comprise a velocity, volume flow rate and/or mass flow rate. The control command can comprise a command for controlling an agitator, e.g. an angular velocity or frequency. The control command can comprise or a command for controlling a temperature control unit, e.g. a heater and/or cooler, and/or a cooling or heating temperature.

A characteristic property of the mixture and/or the fluid flow can be determined when or after carrying out the simulation. In some embodiments, the characteristic property can be or comprise at least one of a mixing fraction, a species concentration, a characteristic mixing time, a characteristic residence time and/or a mixing progress. The characteristic property may be or comprise a characteristic property used for optimizing the mixing reactor geometry.

The characteristic property may be or comprise one or more statistical quantities, such as e.g. an average, variance, or higher moment. It may be provided that the statistical quantity may be an ensemble average, a time average, and/or a density-weighted average, and/or a statistical quantity evaluated and/or computed in space and/or time. In some embodiments, the characteristic property may be computed at or for at least one, multiple or all grid points. The characteristic property may be or comprise a scalar quantity. The characteristic property may be or comprise a directional quantity, such as e.g. a vector or a tensor of higher order.

The control command may be generated and/or updated such that the characteristic property may be close to a target value and/or deviate only by less than a given value form a target value. The control command may be generated and/or updated such that the flow and/or mixing in the (physical) mixing reactor 10 controlled by the control command is equal to, or at least close enough to, the simulated floe and/or mixing.

In some embodiments, the method for controlling a mixing reactor may comprise iterating at least one, several or all steps, such as e.g. simulating and generating and/or adapting the control command.

In some embodiments, the control command may be modeled at least partially or completely by choosing suitable boundary conditions for the simulation.

For example, the control command may comprise a mean flow velocity, volume flow rate or mass flow rate of an inflow, e.g. inflow 70 and/or 80, such that a specific and/or desired mixing is achieved in the mixing reactor 10. Alternatively or additionally, the control command may comprise heating a wall of the mixing reactor 10 with a temperature control unit 110, such that the wall may have a specific and/or desired temperature, which may facilitate or be necessary for mixing. These control commands are only exemplary.

The control command may vary over time, and/or depend on time. For instance, a mean velocity of an inflow (e.g. inflow 70 and/or 80) and/or its mass flow rate or volume flow rate, may vary over time.

The method may comprise measuring a fluid property. The fluid property can e.g. be or comprise one or more of a velocity and/or a species concentration. The fluid property can be measured at or nearby at least one measure point in the mixing reactor 10. For instance, the fluid property can be measured by a sensor 100. The measured fluid property can be compared with a corresponding fluid property obtained from the simulation. For instance, the fluid property obtained from the simulation can be determined at one or several grid points 30 of the computational grid 20, which corresponds to the (physical) measure point of the mixing reactor 10 and/or the position of a corresponding sensor 100. The control command can be generated, adapted and/or modified based on the comparison. For instance, the control command can be

generated, adapted and/or modified such that the measured physical quantity, e.g. the physical quantity as measured by the sensor 100, is equal to or close enough to the physical quantity from the simulation.

In some embodiments, the control command can be generated and/or updated while or when mixing the at least two species in the mixing reactor. In some embodiments, it may be provided that the method for controlling the mixing reactor is carried out "in-line", i.e. while or when operating the mixing reactor. Alternatively or additionally, the control command may be generated and/or updated before operating the mixing reactor.

It may be provided that after generating and/or updating the control command, the simulation of the mixing can be repeated and/or the method can be carried out iteratively When repeating the simulation, the distribution function can be computed taking the generated and/or updated control command into account. For instance, when repeating the simulation, the distribution function can be computed taking e.g. forces, flow velocities and/or boundary conditions imposed and/or exerted by the control command into account.

The mixing reactor can be controlled by the control command. A control unit can be configured to control the mixing reactor 10 as instructed via the control command. In some embodiments, the control unit can be configured to carry out the method for controlling the mixing reactor.

The invention further relates to a data processing device comprising a processor configured to perform the steps of the method for simulating fluid flow according to the invention. The invention further relates to a data processing device comprising a processor configured to perform the steps of the method for designing a mixing reactor according to the invention. The invention further relates to a data processing device comprising a processor configured to perform the steps of the method for controlling a mixing reactor according to the invention.

The data processing device may be or comprise a control unit, such as a control unit controlling the mixing reactor. Alternatively or additionally, the data processing device may be or comprise a general purpose computer and/or computer device, such as a e.g. a distributed computing system, a cloud computing system, a computer cluster, a distributed workstation, a PC, a laptop, a tablet, a smart phone, a server, or the like.

The invention further relates to a computer program comprising instructions which, when the program is executed by a computer, cause the computer to carry out the steps of the method for simulating fluid flow according to the invention. The invention further relates to a computer program comprising instructions which, when the program is executed by a computer, cause the computer to carry out the steps of the method for designing a mixing reactor according to the invention. The invention further relates to a computer program comprising instructions which, when the program is executed by a computer, cause the computer to carry out the steps of the method for controlling a mixing reactor according to the invention.

The program may run in parallel, and/or use multiple CPUs, computer cores and/or nodes.

The invention further relates to a computer-readable medium comprising instructions which, when executed by a computer, cause the computer to carry out the steps of the method for simulating fluid flow according to the invention. The invention further relates to a computer-readable medium comprising instructions which, when executed by a computer, cause the computer to carry out the steps of the method for designing a mixing reactor according to the invention. The invention further relates to a computer-readable medium comprising instructions which, when executed by a computer, cause the computer to carry out the steps of the method for controlling a mixing reactor according to the invention.

The computer-readable medium may be or comprise a disc, such as floppy disc, compact disc (CD), digital versatile disc (DVD), blu ray or the like, a memory, e.g. flash, EEPROM, RAM or the like, hard disc drive, optical or magnetic storage media, or the like.

The features as disclosed in the claims, the specification and the figures may be relevant for the realization of the invention, individually or in any combination.

## List of reference numerals

1 fluid particle
2 tensor
3 tensor train
4 quantized tensor train
5 tensor train core
6 velocity direction

10 mixing reactor
20 computational grid
30 grid point
40 inlet

| | |
|---|---|
| 50 | outlet |
| 60 | sensor |
| 70 | inflow |
| 80 | inflow |
| 90 | outflow |
| 100 | sensor |
| 110 | temperature control unit |

| | |
|---|---|
| $A_I$ | inflow cross-section |
| $A_O$ | outflow cross-section |

| | |
|---|---|
| $\Delta x$ | grid distance |
| L | mixing length |

## Claims

**1.** A computer-implemented method for simulating fluid flow, wherein a computational grid (20) having grid points (30) is provided for computing a distribution function (f, 2) of the fluid flow and/or a distribution function (f, 2) of a mixture of at least two species at the grid points (30),
the method comprising a Lattice Boltzmann method having:

- a collision step, wherein at each grid point (30) a difference of the distribution function (f) to an equilibrium solution ($f^{eq}$) is computed; and
- a streaming step, wherein the distribution function (f) at each grid point (30) is updated according to the streaming step;

and/or the method comprising: solving a Lattice Boltzmann equation for the distribution function (f, 2), **characterized in that** the distribution function (f, 2) is represented in a tensor-train format, and/or all operations carried out for computing the distribution function (f, 2) are carried out with the distribution function (f, 2) in the tensor-train format, the distribution function (f, 2) and/or the tensor train format comprising at least one tensor train (3), the tensor train (3) having at least one tensor train core (4).

**2.** The computer-implemented method of claim 1, wherein the tensor train (3) is or comprises a quantized tensor train.

**3.** The computer-implemented method of any of the preceding claims, wherein the tensor-train (3) has tensor train cores (5) respectively corresponding to grid coordinates and/or velocity components of the flow.

**4.** The computer-implemented method of any of the preceding claims, wherein the tensor-train (5) has at least one tensor train core (5) corresponding to the species, and/or wherein for each species a tensor-train is provided.

**5.** A computer-implemented method for designing a mixing reactor (10) for mixing at least two species, wherein the method comprises providing an initial mixing reactor geometry having a computational grid (20) with grid points (30), the method comprising the steps:

a) simulating a mixing of at least two species, and/or a flow in and/or through the mixing reactor (10), via the method of any of the preceding claims, and determining a characteristic property of the mixture and/or fluid flow, optionally at least one of a mixing fraction, a species concentration, a characteristic mixing time, a characteristic residence time and/or a mixing progress;
b) modifying the mixing reactor geometry and/or grid points (30), repeating the simulating using the modified mixing reactor geometry and/or grid points (30), and determining the characteristic property of the mixture;
c) comparing the characteristic property to a target value of the characteristic property;
d) iteratively repeating the steps b) and c), and optimizing the mixing reactor geometry and/or the computational grid (20), such that the mixing reactor geometry and/or the computational grid (20) is optimized with respect to the characteristic property, and/or until a given accuracy of the characteristic property to the target value is achieved.

**6.** The method of claim 5, wherein modifying the mixing reactor geometry comprises moving a boundary and/or changing the shape of the computational grid (20), and/or wherein modifying the grid points (30) comprises adding or removing grid points (30) from the computational grid (20).

**7.** A mixing reactor (10) for mixing at least two species, the mixing reactor (10) having a mixing reactor geometry, optionally an interior geometry, which is determined via the method of any of the preceding claims 5 to 6.

**8.** A computer-implemented method for controlling a mixing reactor (10), the method comprising:

- simulating a mixing of at least two species, and/or a flow, in and/or through the mixing reactor (10), via the method of any of the preceding claims 1 to 4, wherein the computational grid (20) corresponds to a geometry of the mixing reactor (10);
- generating and/or updating at least one control command for the mixing reactor (10) based on the simulation.

**9.** The method of claim 8, wherein the control command comprises one or more of a command for controlling an inflow (70, 80) of at least one flow into the mixing reactor (10) and/or an outflow (90) of at least one flow out of the reactor, optionally a velocity, volume flow rate and/or mass flow rate, a command for controlling an agitator, optionally an angular velocity or frequency, and/or a command for controlling a temperature control unit (110), optionally a heater and/or cooler, optionally a cooling or heating temperature.

**10.** The method of any of claims 8 to 9, wherein the method comprises measuring a fluid property, optionally a velocity and/or a species concentration, at at least one measure point in the mixing reactor (10), optionally with a sensor (110), and comparing the measured fluid property with a corresponding fluid property obtained from the simulation, wherein the control command is generated, adapted and/or modified based on the comparison.

**11.** The method of any of claim 8 to 10, wherein the control command is generated and/or updated while or when mixing the at least two species in the mixing reactor (10), wherein optionally the mixing reactor (10) is controlled by the control command.

**12.** The method of any of claims 8 to 11, wherein after generating and/or updating the control command, the simulation of the mixing is repeated, wherein when repeating the simulation, the distribution function (f, 2) is computed taking the generated and/or updated control command, optionally forces, flow velocities and/or boundary conditions imposed and/or exerted by the control command, into account.

**13.** A data processing device comprising a processor configured to perform the steps of the method of any of the preceding claims 1 to 6 and/or 8 to 12.

**14.** A computer program or computer program product comprising instructions which, when the program is executed by a computer, cause the computer to carry out the steps of the method of any of the preceding claims 1 to 6 and/or 8 to 12.

**15.** A computer-readable medium comprising instructions which, when executed by a computer, cause the computer to carry out the steps of the method of any of the preceding claims 1 to 6 and/or 8 to 12.

Fig. 1

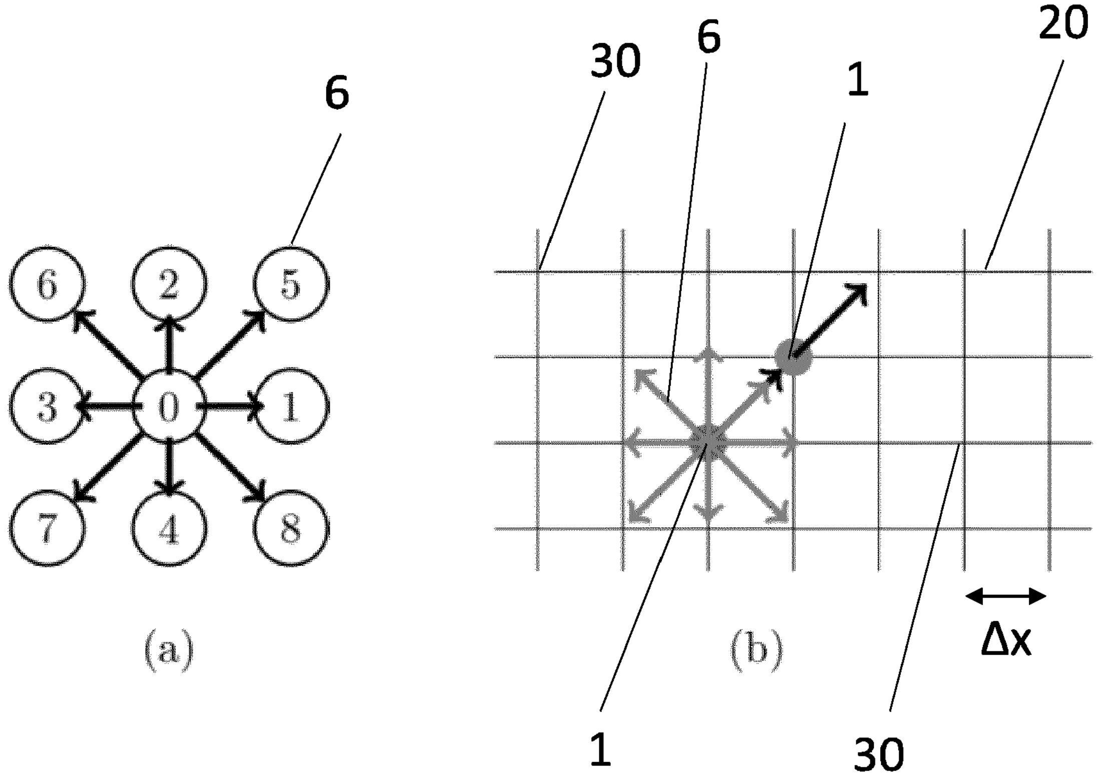

Fig. 2

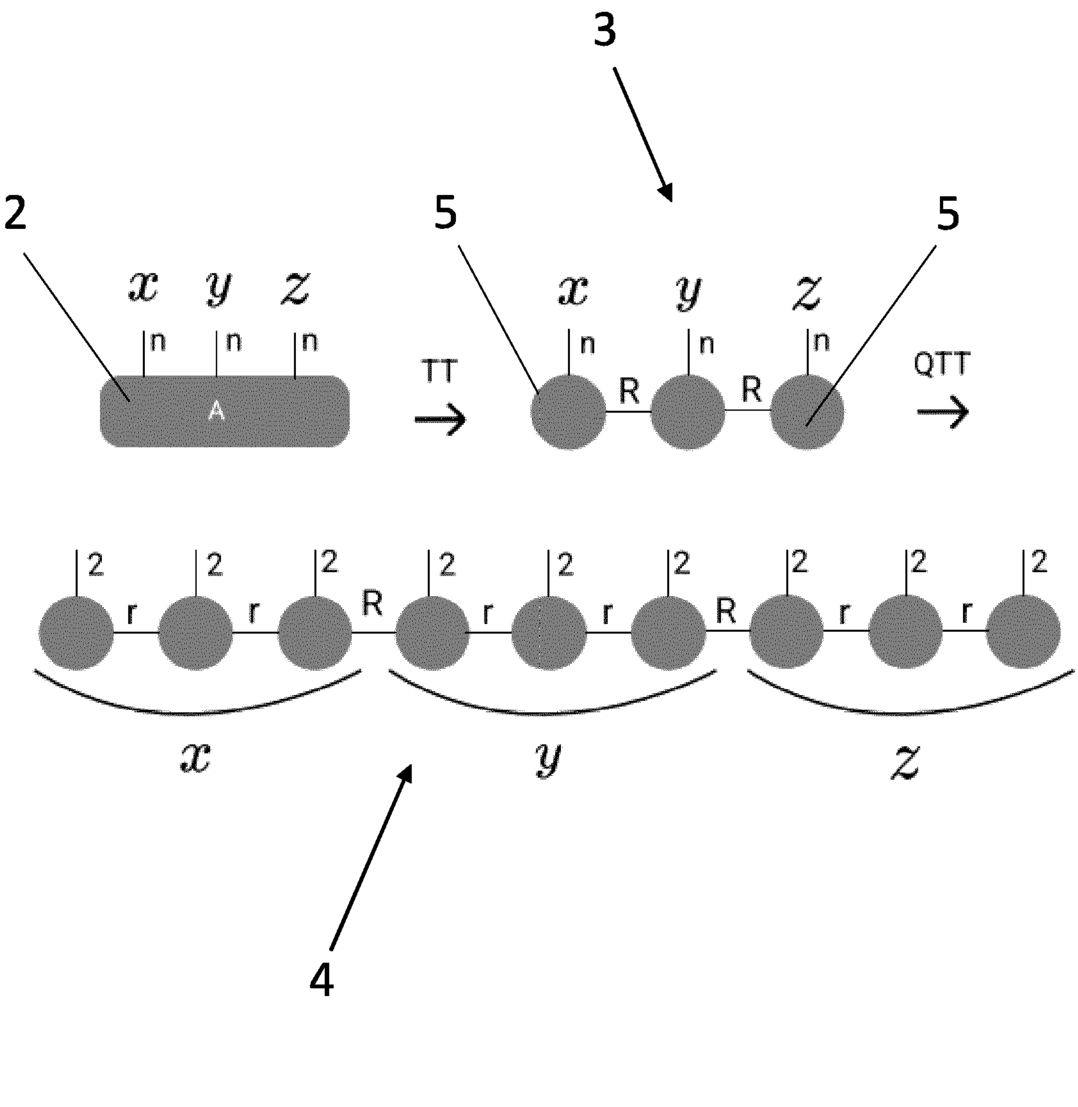

Fig. 3

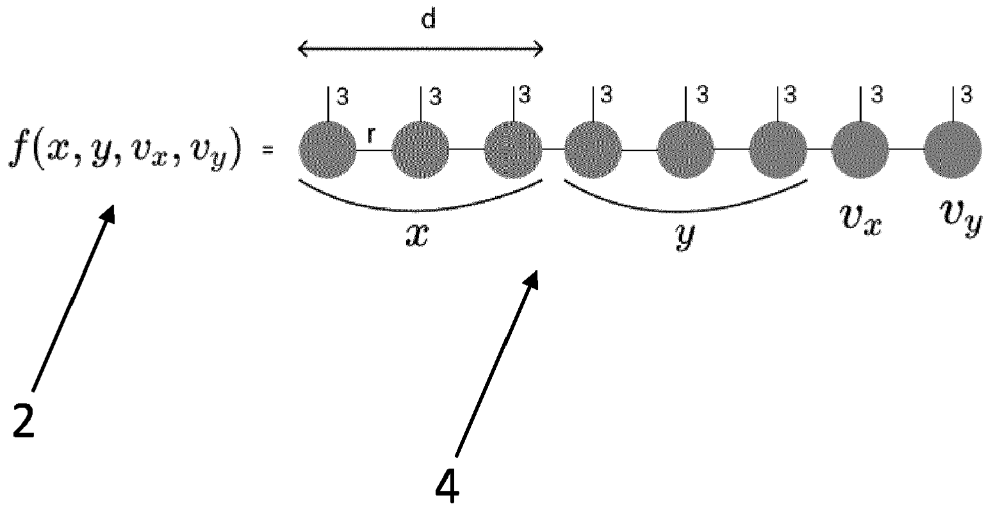


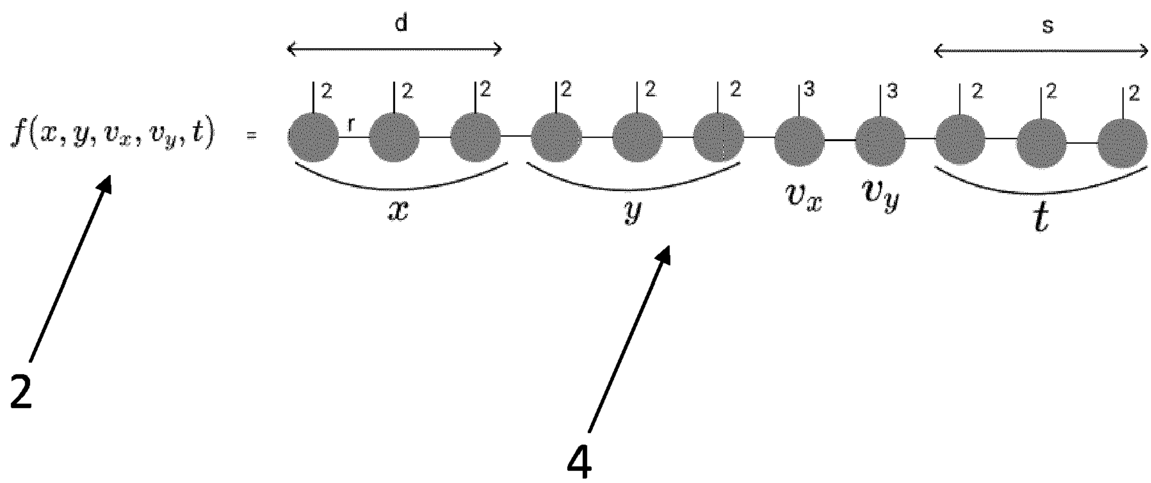

Fig. 4

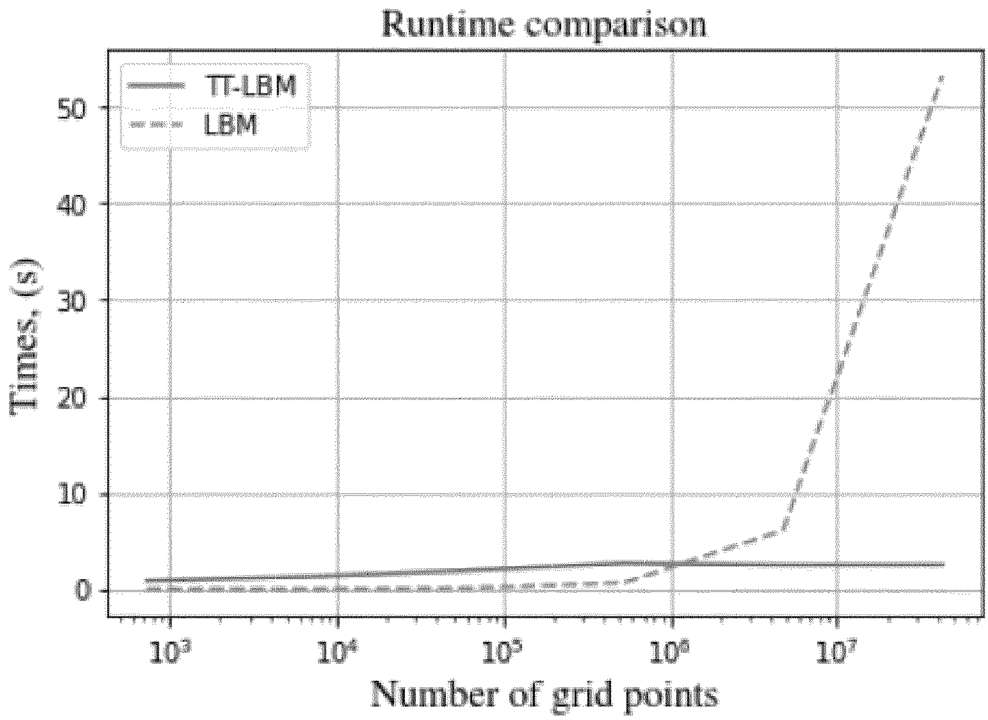
Runtime comparison
TT-LBM
LBM
Times, (s)
0
10
20
30
40
50
$10^3$
$10^4$
$10^5$
$10^6$
$10^7$
Number of grid points

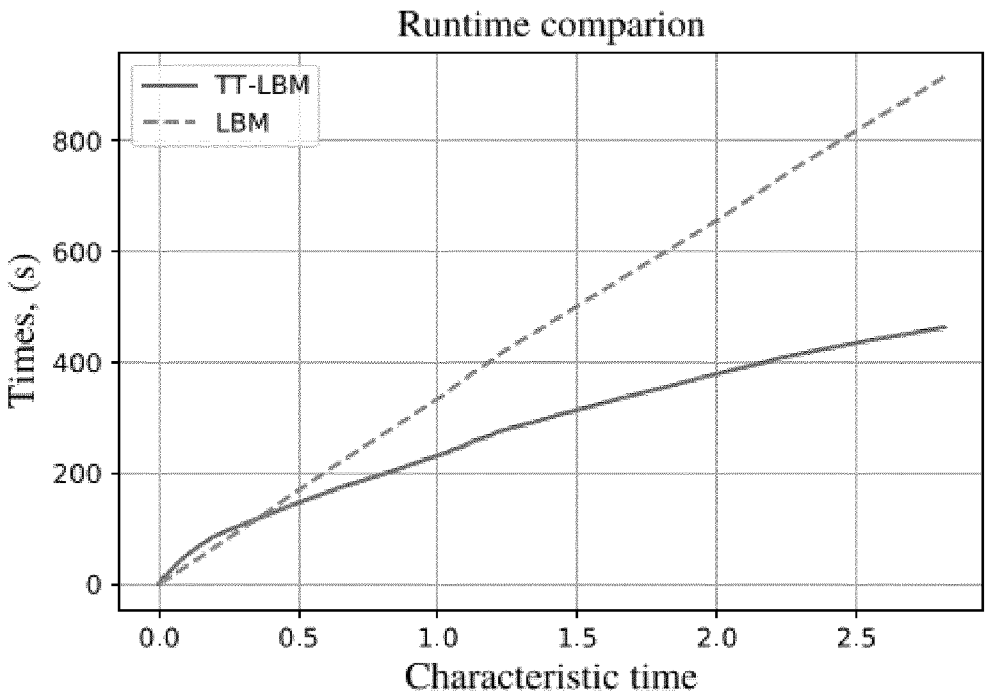
Runtime comparion
TT-LBM
LBM
Times, (s)
0
200
400
600
800
0.0
0.5
1.0
1.5
2.0
2.5
Characteristic time

Fig. 5

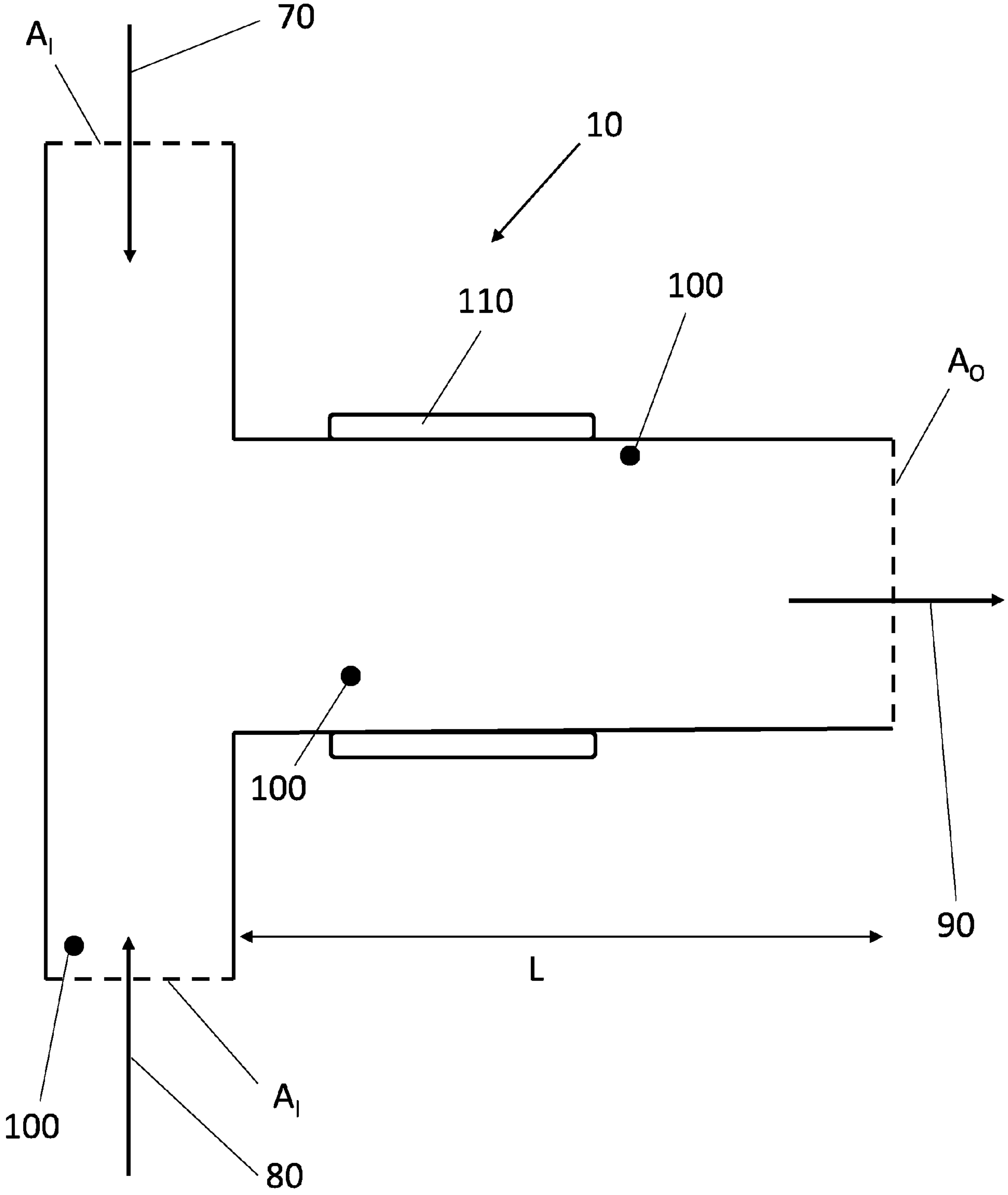

Fig. 6

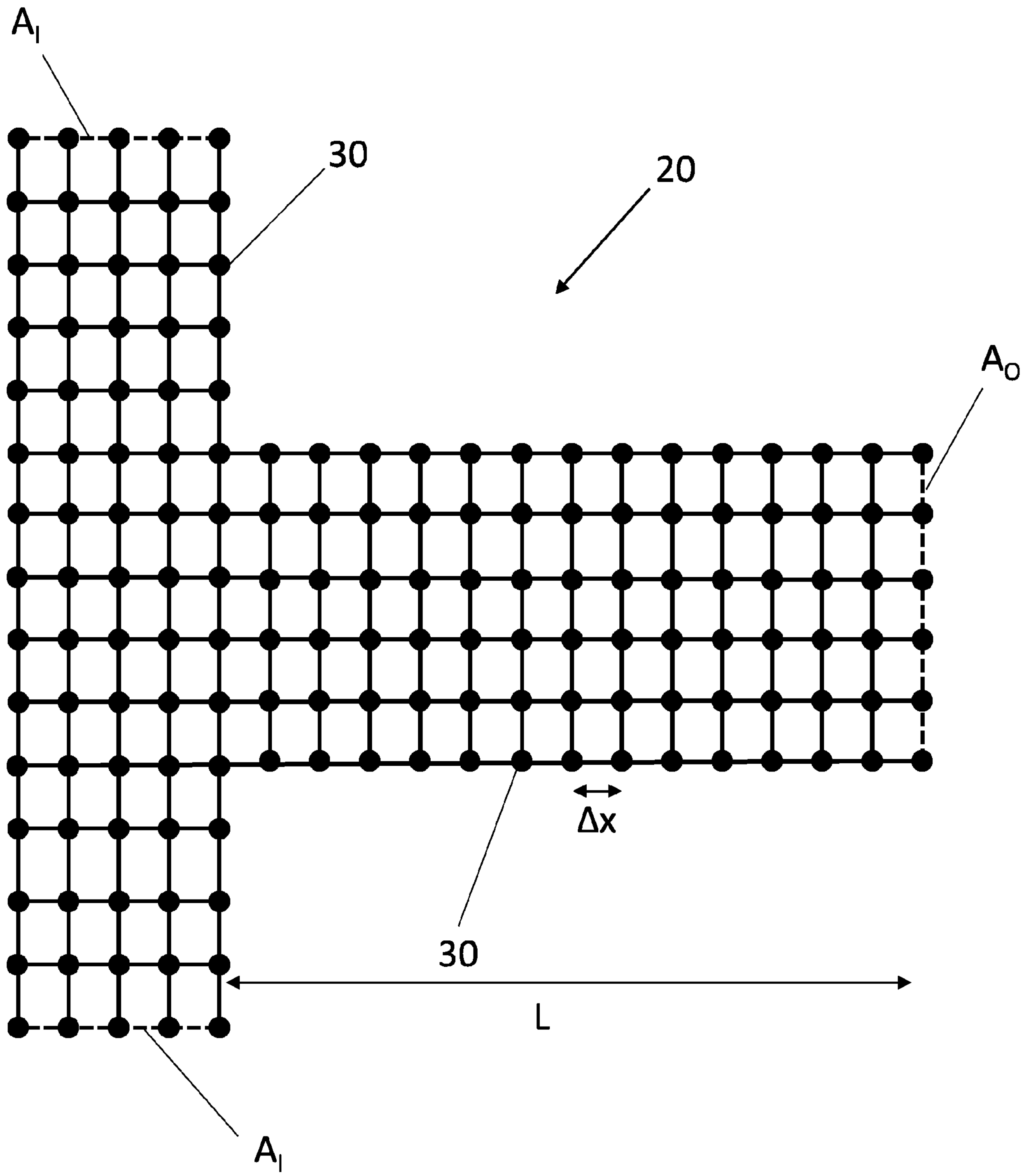

Fig. 7

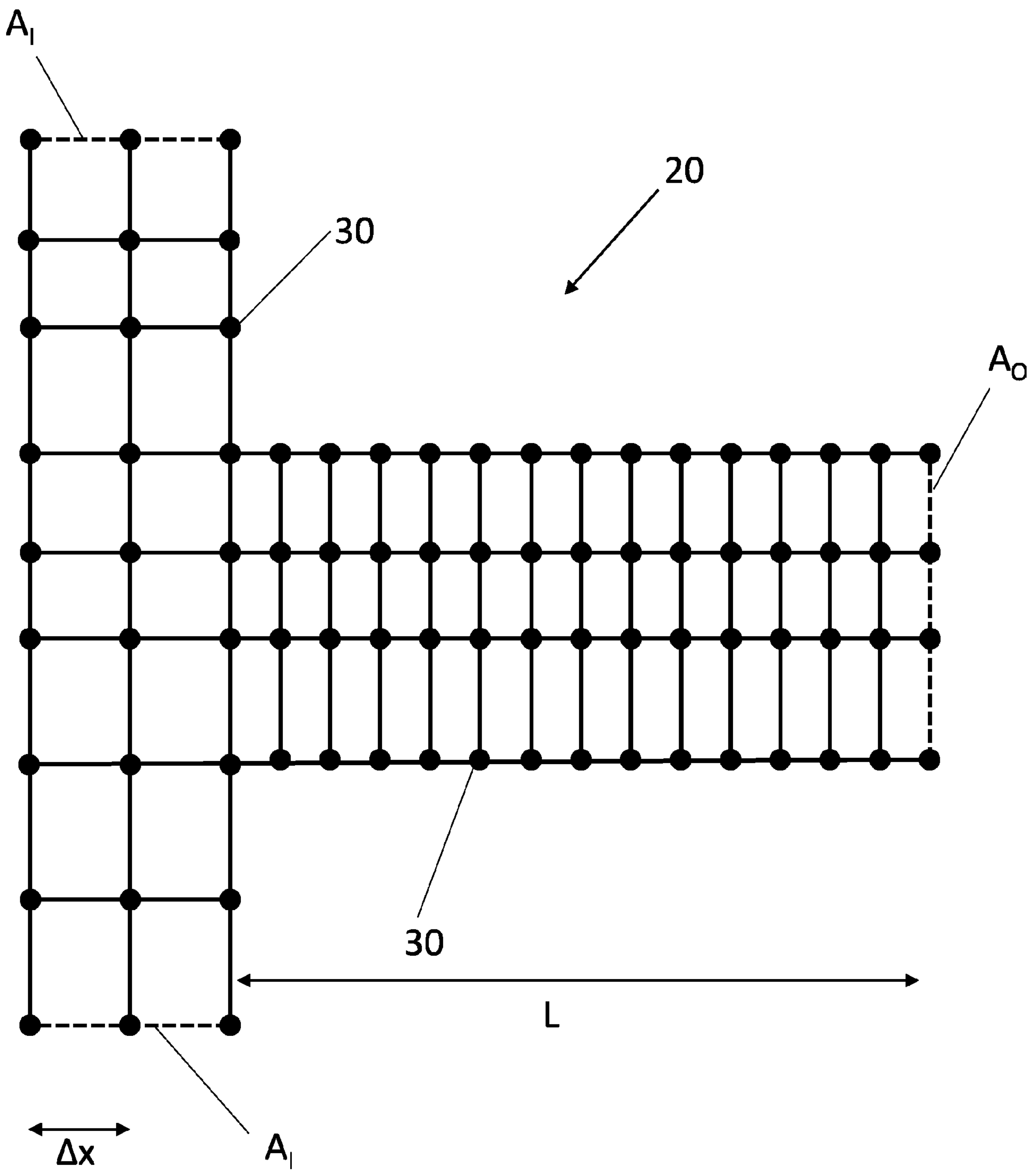

Fig. 8

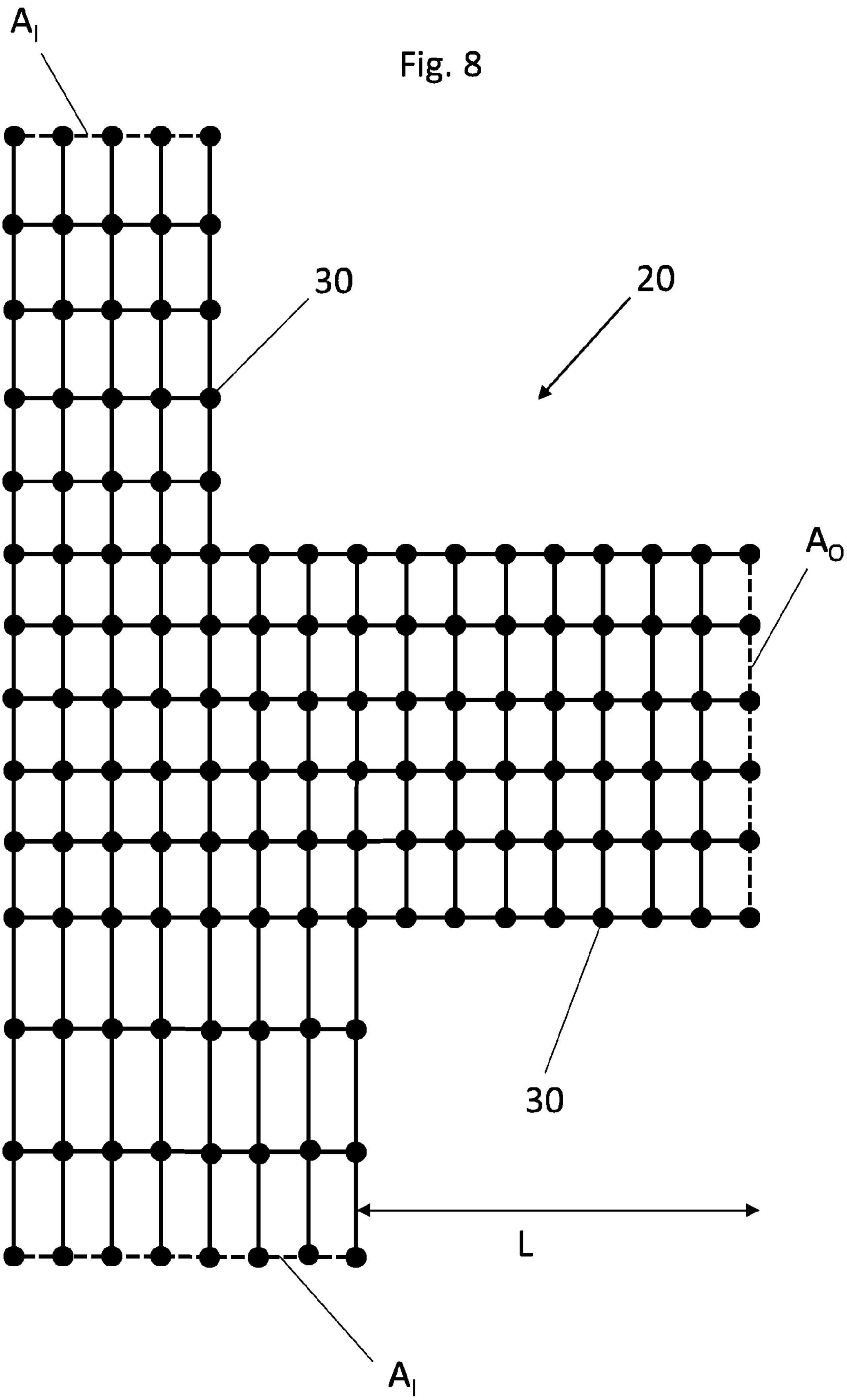

Europäisches Patentamt
European Patent Office
Office européen des brevets

# EUROPEAN SEARCH REPORT

Application Number
EP 23 21 8407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EHSAN MEHRABI GOHARI ET AL: "Hydrodynamic simulation of a liquid-solid fluidized bed using Lattice Boltzmann and smoothed profile methods", ASIA-PACIFIC JOURNAL OF CHEMICAL ENGINEERING, JOHN WILEY & SONS LTD, US, vol. 12, no. 2, 10 January 2017 (2017-01-10), pages 196-211, XP072445138, ISSN: 1932-2135, DOI: 10.1002/APJ.2065 * abstract * * pg. 197, col. 1, par. 2 pg. 197, col. 2, par. 2, last paragraph pg. 199, col. 1 pg. 201, col. 1, par. 5; figures 2, 5 * * the whole document * ----- | 1-15 | INV. G06F30/17 G06F30/28 G16C20/10 ADD. G06F111/08 G06F111/10 G06F113/08 |
| X | SIRASITTHICHOKE CHADAKARN ET AL: "Power number and hydrodynamic characterization of a stirred vessel equipped with a Retreat-Blade Impeller and different types of pharmaceutical single baffles", CHEMICAL ENGINEERING SCIENCE, OXFORD, GB, vol. 257, 13 May 2022 (2022-05-13), XP087098470, ISSN: 0009-2509, DOI: 10.1016/J.CES.2022.117725 [retrieved on 2022-05-13] * abstract * * section 4.3 * * figure 1 * * the whole document * ----- | 1-15 | TECHNICAL FIELDS SEARCHED (IPC) G06F G16C |

-/--

The present search report has been drawn up for all claims

1

| Place of search | Date of completion of the search | Examiner |
|---|---|---|
| Munich | 31 May 2024 | Brandiska, Pavlina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 21 8407

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EGOR KORNEV ET AL: "Numerical solution of the incompressible Navier-Stokes equations for chemical mixers via quantum-inspired Tensor Train Finite Element Method", ARXIV.ORG, CORNELL UNIVERSITY LIBRARY, 201 OLIN LIBRARY CORNELL UNIVERSITY ITHACA, NY 14853, 18 May 2023 (2023-05-18), XP091511360, * abstract * * section III.B * * figures 1, 2, 4, 5, 7 * * the whole document * ----- | 1-15 | |
| X | NIKITA BELOKONEV ET AL: "Optimization of chemical mixers design via tensor trains and quantum computing", ARXIV.ORG, CORNELL UNIVERSITY LIBRARY, 201 OLIN LIBRARY CORNELL UNIVERSITY ITHACA, NY 14853, 24 April 2023 (2023-04-24), XP091493481, * abstract * * figures 1, 3, 7 * * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 31 May 2024 | Brandiska, Pavlina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHEN** ; **DOOLEN**. Lattice boltzmann method for fluid flows. *Annual review of fluid mechanics*, 1998, vol. 30 (1), 329-364
- **BENZI** ; **SUCCI** ; **VERGASSOLA**. The lattice Boltzmann equation: theory and applications. *Physics reports (Review Section of Physics Letters)*, 1992, vol. 222 (3), 145-197
- **OSELEDETS** ; **TYRTYSHNIKOV**. Tt-cross approximation for multidimensional arrays. *Linear Algebra and its Applications*, 2010, vol. 432 (1), 70-88
- **MELVEN** ; **THIES** ; **BASERMANN**. Performance of the low-rank tt-svd for large dense tensors on modern multicore cpus. *SIAM Journal on Scientific Computing*, 2022, vol. 44 (4), C287-C309
- **DOLGOV** ; **KHOROMSKIJ** ; **OSELEDETS**. Fast solution of parabolic problems in the tensor train/-quantized tensor train format with initial application to the fokker-planck equation. *SIAM Journal on Scientific Computing*, 2012, vol. 34 (6), A3016-A3038
- **GOURIANOV** ; **LUBASCH** ; **DOLGOV** ; **VAN DEN BERG** ; **BABAEE** ; **GIVI** ; **KIFFNER** ; **JAKSCH**. A quantum-inspired approach to exploit turbulence structures. *Nature Computational Science*, 2022, vol. 2 (1), 30-37
- **ROHRBACH** ; **DOLGOV** ; **GRASEDYCK** ; **SCHEICHL**. Rank bounds for approximating gaussian densities in the tensor-train format. *arX1υ:2001.08187*, 2020
- **OSELEDETS**. Tensor-train decomposition. *SIAM J. Scientific Computing*, January 2011, vol. 33, 2295-2317
- **DOLGOV** ; **SAVOSTYANOV**. Alternating minimal energy methods for linear systems in higher dimensions. *SIAM Journal on Scientific Computing*, September 2014, vol. 36 (5), 1-24